# EUROPEAN PATENT APPLICATION

(11) **EP 3 456 387 A1**
(43) Date of publication of application: **20.03.2019**
(21) Application number: 18184248.5
(22) Date of filing: 18.07.2018
(51) Int. Cl.: A61Q 11/00, A61K 8/81

(54) **ORAL CARE COMPOSITIONS**

(30) Priority: 18.09.2017 EP 17191533
(71) Applicant: Unilever PLC, a company registered in England and Wales under company no. 41424 of, London EC4Y 0DY (GB); Unilever N.V., 3013 AL Rotterdam (NL)
(72) Inventor: JOINER, Andrew, Wirral, Merseyside CH63 3JW (GB); LUO, Wen, Wirral, Merseyside CH63 3JW (GB)
(74) Representative: Tansley, Sally Elizabeth

(57) **Abstract**

Use of a colourant having a hue angle, *h*, in the CIELAB system of from 220 to 320 degrees, for enhancing tooth shine.

## Description

The present invention relates to oral care compositions that enhance the shine of the teeth.

The object of the invention is to deliver improved tooth shine.

### Description of the Invention

In a first aspect of the present invention relates to the use of a colourant having a hue angle, h, in the CIELAB system of from 220 to 320 degrees, for enhancing tooth shine.

### Detailed Description of the Invention

Tooth shine is a parameter that relates to the shiny appearance of a tooth. It relates to the whole tooth surface.

The present application is concerned with improved tooth shine.

The colourants used to improve shine are preferably a dye or a pigment or a mixture thereof.

A pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37°C. As a reasonable approximation, the relevant medium may be considered to be water and the relevant temperature to be 25°C.

The total amount of colourant, in the composition is preferably from 0.0001 to 0.1wt%, preferably from 0.0005 to 0.05wt%, and more preferably from 0.001 to 0.01wt% by weight. The level of pigment is preferably higher in the composition than the level of dye. Preferably, the level of pigment is from 0.01 to 0.3%, preferably from 0.02 to 0.1%, and more preferably from 0.03 to 0.08% by weight. The percentage weights refer to the total amount of active present and exclude any carriers that may be present.

The pigment may be uniformly spread throughout the composition or it may be dispersed in a second phase such as a stripe or other co-extruded second phase. Such "dual phase" compositions have the advantage that the phases may be differently coloured, presenting a more visually attractive product to the consumer.

Preferably, the pigment is violet or blue, more preferably one of those listed in the Colour Index International. These pigments are listed as pigment violet 1 through to pigment violet 56 and pigment blue 1 through 83.

The preferred pigment violets are pigment violet 1, 1:1, 1:2, 2, 3, 5:1, 13, 19, 23, 25, 27, 31, 32, 37, 39, 42, 44 and 50.

The preferred pigment blues are pigment blue 1, 2, 9, 10, 14, 15, 15:1, 15:2, 15:3, 15:4, 15:6 16, 18, 19, 24:1, 25, 56, 60, 61, 62 and 66.

Other suitable pigments are pigment ultramarine blue and ultramarine violet.

The pigment should have a hue angle, h, in the CIELAB system of from 220 to 320 degrees most preferably between 250 and 290 degrees. A detailed description of hue angle may be found on p57 of Colour Chemistry 3rd edition by H. Zollinger published by Wiley-VCH. While the preferred single pigments are blue or violet, the same effect may be achieved through mixing pigments outside of this h range; for example, such a hue angle may also be obtained by mixing a red and green-blue pigment, to yield a blue or violet shaded pigment, or a green and blue pigment, to yield a blue-green shaded pigment.

Preferably, the pigment is Pigment Blue 15, more preferably Pigment Blue 15:1, 15:2, 15:3, 15:4, 15:5 or 15:6, most preferably 15:1.

Preferably, the pigment is capable of reflecting sufficient light such that the treated tooth is perceivably whiter than its initial colour. Preferably, the pigment is coloured such that its natural colour is within the violet-red to green-blue colour, preferably from violet to blue.

If the colourant comprises a dye it is preferable that the dye is a blue dye, more preferably FD&C Blue 1 or Patent Blue V.

Preferably the composition comprises a polymer having a molecular weight of 200,000 or greater.

Preferred deposition aids are Gantrez® type polymers, high molecular weight PEGs, and high molecular weight cellulose ethers. Particularly preferred deposition aids are Gantrez® type polymers and high molecular weight PEGs. Especially preferred deposition aids are Gantrez® type polymers.

Gantrez® type polymers are co-polymers of maleic anhydride with methyl vinylether, in which the anhydride moiety may be in a partially or fully hydrolysed or alcoholysed form. Gantrez® polymers themselves are available from ISP Inc. Suitable Gantrez® polymers are:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Gantrez® type polymers in which the anhydride moiety is fully hydrolysed are preferred. These may be thought of as co-polymers of maleic acid and methyl vinylether. Particularly preferred co-polymers of maleic acid and methyl vinylether have a molecular weight of 1,000,000 or greater and an especially preferred material is Gantrez S-97.

High molecular weight PEGs are poly(ethyleneglycol) polymers having a molecular weight of 1,000,000 or greater, preferably 2,000,000 or greater. A preferred high molecular weight PEG is Polyox 60K, a polymer having a molecular weight of approximately 2,000,000.

High molecular weight cellulose ethers have a molecular weight sufficient to give a 2% aqueous solution of the polymer a viscosity of 1,000 mPa.s or greater, preferably 2,000 mPa.s or greater, and more preferably 3,000 mPa.s or greater. Viscosities are measured at 20°C and 10 s⁻¹ using an Ubbelohde viscometer. Preferred high molecular weight cellulose ethers are hydroxypropylcelluloses, methylcelluloses, and hydroxyethylcelluloses. Particularly preferred high molecular weight cellulose ethers are hydroxypropyl-methylcelluoses.

The polymer is incorporated into the composition at preferably from 0.01 to 10%, more preferably at from 0.05 to 5%, and most preferably at from 0.1 to 1% by weight.

Preferably, the oral care composition comprises water, thickener, surfactant and abrasive. Suitable thickeners include silicas and calcium carbonate. The preferred thickener is silica. Suitable surfactants include the alkali-metal alkyl sulphate surfactants such as the sodium alkyl sulphates, the most preferred being sodium laurylsulphate.

Preferred abrasive materials include silicas, aluminas, calcium carbonates, dicalcium phosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. The most preferred abrasives are calcium carbonate and silica, especially silica.

In addition to the colourant compositions of the invention may comprise a further whitening agent, suitable whitening agents include titanium dioxide, and a pearlescer, preferably mica.

The oral care compositions according to the invention may comprise further ingredients which are common in the art, such as: antimicrobial agents, e.g. Triclosan, chlorhexidine, copper, zinc, and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol); anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
humectants such as glycerol, sorbitol, propyleneglycol, xylitol, lactitol etc.;
binders and thickeners such as sodium carboxymethyl-cellulose, xanthan gum, gum arabic etc. as well as synthetic polymers such as polyacrylates and carboxyvinyl polymers such as Carbopol®;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral care compositions may be in any form common in the art, preferred forms are toothpastes, gels and whitening serums/gels/varnishes.

The invention will now be illustrated by the following non-limiting Examples. Examples of the invention are illustrated by a number, comparative Examples by a letter.

### Examples

The formulations below were chosen for the clinical because they give different modes of action to impact shine index, as follows:
Example 1 delivers a polymer film to the tooth surface which will give a gloss boost, plus contains blue dye and micas.

Professional polish: will deliver increase in gloss through a polishing/abrasive action, but no improvement in whiteness.

Example 2: will deliver an improvement in WIO but unlikely to improve gloss intensity/area.

Example A non-whitening TP: should deliver no significant improvement in WIO or Gloss area/intensity.

The clinical results below and detailed analysis confirm these modes of action.

**Table 1**

| **Ingredient Name** | **Example 1 Wt%** |
|---|---|
| Bentone gel | 12 |
| Mica/titanium dioxide | 1.5 |
| Ethyl acetate | 20 |
| Acrylates/T-octylpropenamide copolymer | 10 |
| Blue patent v | 0.00125 |
| Isopropyl alcohol and minors | To 100 |

**Table 2**

| **Chemical Name** | **INCI Name** | **Example A %w/w** | **Example 2 %w/w** |
|---|---|---|---|
| Sorbitol Liquid | Sorbitol | 45.00 | 65 |
| | | | |
| Hydrated Silica | Hydrated Silica | 17.5 | 14.2 |
| | Gantrez S97 | | 1.0 |
| PEG-32 | PEG-32 | 5.00 | 1.5 |
| | Polyox | | 1.0 |
| PEG-30 Stearyl ether | Steareth 30 | | 1.8 |
| Sodium Lauryl Sulphate | | 1.5 | |
| Titanium Dioxide | CI77891 | 1.00 | |
| SCMC 9H4XF | Cellulose Gum | 0.63 | 0.52 |
| Sodium Fluoride | Sodium Fluoride | 0.32 | 0.32 |
| Monosodium Phosphate | Monosodium Phosphate | 0.1 | 0.5 |
| Blue covarine W6795 | | | 0.19 |
| Water and minors | | T0 100 | To 100 |

A clinical study with human volunteers was conducted. At each test visit, subjects had digital images taken of their teeth using a Gloss Imaging System before and immediately after using one of the study products (Tables 1 and 2). Subjects either brushed their teeth with one of the toothpastes followed by rinsing or the study hygienist applied the paint-on tooth lacquer or conducted a prophylaxis polishing procedure on the anterior teeth. Tooth Whiteness (WIO) and Tooth Shine Index values were determined from the digital images (see equation) and changes in WIO and Tooth Shine Index from baseline were calculated (Table 3).

On the final visit, subjects viewed their own tooth digital images and assessed them to see if any changes in the measured shine and whiteness parameters can be observed subjectively.

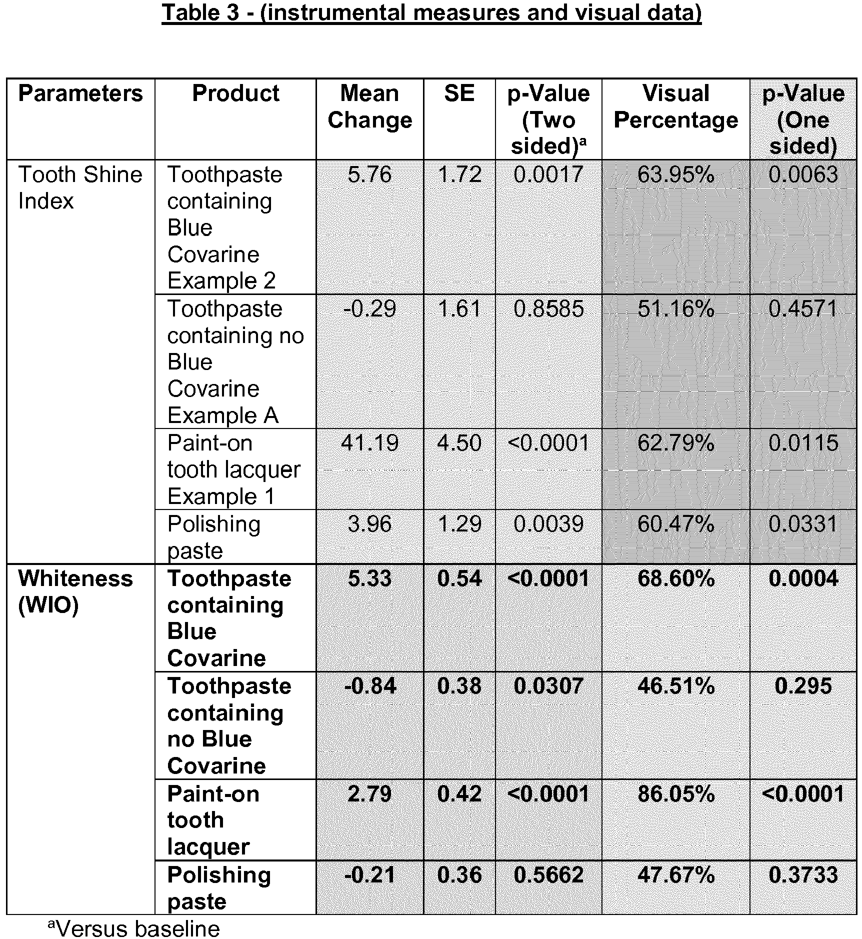

The instrumental measured mean changes of tooth shine and whiteness for the four products are listed above (Table 3), the consumer perception results are listed in the last two columns (percentages of observations when the post brushing images were judged as shinier/whiter). The results show that the polishing paste gave an increase in measurable tooth shine but no significant increase in WIO. The paint-on tooth lacquer gave a significant increase in tooth shine and WIO. The toothpaste containing no Blue Covarine gave no significant increase in tooth shine and WIO. The toothpaste containing Blue Covarine gave a significant increase in tooth shine and WIO. Where there is a significant increase in WIO and tooth shine, it is perceivable by the consumer. In addition, the above table demonstrates that toothpastes with a blue colourant are seen to improve the consumers' perception of shine.

## Claims

1. Use of a colourant having a hue angle, h, in the CIELAB system of from 220 to 320 degrees, for enhancing tooth shine.

2. Use according to claim 1 in which the colourant has a hue angle, h, in the CIELAB system of from 250 to 290 degrees.

3. Use according to any preceding claim in which the colourant comprises blue or green colourant.

4. Use according to any preceding claim in which the colorant is a pigment.

5. Use according to claim 4 in which the level of pigment is from 0.01 to 0.3% by weight of the total composition.

6. Use according to any preceding claim in which the pigment comprises pigment blue 15.

7. Use according to any preceding claim in which the colourant comprises a pigment and a dye.

8. Use according to any preceding claim in which the composition further comprises a polymer having a molecular weight of 200,000 or greater.

9. Use according to claim 7, in which the polymer is selected from the group consisting of Gantrez® type polymers, high molecular weight PEGs, and high molecular weight cellulose ethers.

10. Use according to claim 7 in which the polymer is a co-polymer of maleic acid and methyl vinylether.

11. Use according to any preceding claim in which the composition further comprises a silica based abrasive.
